# FASCICULE DE BREVET EUROPEEN

(11) **EP 2 257 232 B1**
(45) Date de publication et mention de la délivrance du brevet: **07.12.2011**
(21) Numéro de dépôt: 09723813.3
(22) Date de dépôt: 26.03.2009
(51) Int. Cl.: A61B 17/34, A61B 17/88, A61F 2/46, A61M 5/00

(54) **DISPOSITIF POUR L'INJECTION DANS L'ORGANISME D'UN FLUIDE VISQUEUX**
VORRICHTUNG ZUR EINSPRITZUNG EINER VISKÖSEN FLÜSSIGKEIT IN DEN KÖRPER
DEVICE FOR INJECTING A VISCOUS FLUID INTO THE BODY

(30) Priorité: 28.03.2008 FR 0852010
(43) Date de publication de la demande: 08.12.2010
(73) Titulaire: Persat, Jean-Charles, 1239 Collex-bossy (CH)
(72) Inventeur: Persat, Jean-Charles, 1239 Collex-bossy (CH)
(74) Mandataire: Thibault, Jean-Marc
(86) Numéro de dépôt international: PCT/EP2009/053632
(87) Numéro de publication internationale: WO 2009/118397

(56) Documents cités:
- WO-A-2005/051212
- WO-A-2006/125100
- WO-A-2007/036815
- US-A1- 2005 070 912
- US-A1- 2008 065 083

## Description

L'objet de l'invention concerne le domaine technique de l'instrumentation médicale et vise plus précisément un dispositif adapté pour assurer l'injection à pression élevée d'un fluide visqueux dans l'organisme.

L'objet de l'invention trouve une application particulièrement avantageuse dans le domaine de la chirurgie par l'injection et la mise en place sous la forme d'un fluide visqueux, de produits de traitement, de consolidation ou prothétique.

A titre d'exemple non limitatif, le fluide visqueux injecté peut être de nature à assurer une reconstruction osseuse, notamment du rachis, par injection de substituts osseux ou de ciments osseux.

Dans le domaine technique de la reconstruction osseuse, il est connu d'injecter une dose d'un produit visqueux à l'aide d'une seringue qui est destinée à être fixée à l'extrémité d'un vecteur d'injection tel un trocart préalablement installé dans le corps du patient.

A titre d'exemple, la demande de brevet WO 2006/125100 décrit une seringue d'injection d'un fluide visqueux de reconstruction osseuse comportant un corps de seringue rigide munie à une extrémité d'un organe de préhension et sur l'extrémité opposée, d'un prolongateur flexible monté à l'aide d'un raccord. Cette seringue comporte une tige de piston possédant une section droite transversale en forme de croix. Une extrémité de la tige de piston est munie d'un organe de poussée tandis que l'extrémité opposée engagée dans le corps de seringue est équipée d'un joint d'étanchéité.

Dans le domaine d'utilisation de cette seringue, il est à considérer que le fluide injecté présente généralement une viscosité relativement importante et une vitesse de polymérisation importante. Il s'ensuit que l'opération d'injection est relativement difficile à mener à bien et doit être effectuée rapidement. En effet, le praticien doit exercer une force de pression relativement importante sur le piston de la seringue pour assurer l'injection du fluide dans l'organisme. L'effort de poussée important empêche le praticien de pouvoir contrôler au mieux la procédure d'injection du fluide visqueux. De plus, il arrive fréquemment un blocage voire une détérioration de la seringue lors de l'opération d'injection.

L'objet de l'invention vise donc à remédier aux inconvénients de l'état de la technique en proposant un nouveau dispositif permettant d'assurer facilement l'injection sous pression élevée d'un fluide visqueux dans un organisme.

Un autre objet de l'invention et de proposer un dispositif d'injection d'un fluide visqueux, offrant la possibilité d'occuper diverses positions par rapport au vecteur d'injection, pour tenir compte des contraintes d'encombrement autour du site d'injection.

Pour atteindre un tel objectif, le dispositif pour l'injection dans l'organisme d'un fluide visqueux comporte :
- une structure tubulaire délimitant un alésage interne de passage pour le fluide visqueux, comportant à partir d'une première extrémité équipée d'un organe de préhension, une partie rigide contigüe à une partie flexible,
- un piston de poussée engagée dans l'alésage interne, et équipé à une première extrémité d'un organe de poussée et présentant une longueur inférieure à la longueur de la partie rigide.

Selon l'invention :
- la structure tubulaire est un corps tubulaire métallique monobloc s'étendant de la première extrémité à une seconde extrémité munie d'un raccord pour la fixation étanche d'un vecteur d'injection transtissulaire,
- le piston de poussée comporte une tige métallique présentant une section droite transversale constante sur toute sa longueur et complémentaire au jeu de fonctionnement près, à celle de l'alésage du corps tubulaire métallique, la tige métallique présentant à sa seconde extrémité, une face transversale plane de poussée pour le fluide visqueux.

Un autre objet de d'invention est de proposer un dispositif d'injection permettant de maîtriser avec précision le volume du fluide visqueux à injecter.

A cet effet, le dispositif d'injection comporte une tige métallique comportant des indicateurs de volume du fluide injecté.

Avantageusement, la tige métallique comporte une face transversale plane de poussée de surface comprise entre 0,5 et 80 mm² et par exemple de l'ordre de 10 mm².

De préférence, le corps tubulaire métallique comporte une partie flexible dont le diamètre interne est supérieur au plus de 30 % par rapport au diamètre interne du vecteur d'injection transtissulaire.

Avantageusement, le corps tubulaire métallique présente un diamètre interne sensiblement constant sur toute sa longueur.

Selon une variante de réalisation, le corps tubulaire comporte un tube rigide apte à former la partie rigide du corps tubulaire, le tube rigide étant fixé autour d'une chemise flexible dont une partie s'étend en dehors du tube rigide de manière à former la partie flexible du corps tubulaire métallique.

Selon une autre variante de réalisation, le corps tubulaire comporte un tube flexible sur l'extrémité duquel est emmanché un tube rigide dont le diamètre interne détermine le volume du fluide injecté.

Selon une autre variante de réalisation, le corps tubulaire comporte un orifice muni d'un organe de sureté.

Avantageusement, l'organe de sureté est monté mobile pour dégager l'orifice afin de purger le corps tubulaire.

De préférence, le corps tubulaire présente une partie rigide droite et une partie flexible courbe.

Selon une caractéristique de l'invention, le dispositif d'injection comporte une enveloppe tubulaire entourant concentriquement le corps tubulaire métallique sur au moins une partie de sa longueur pour délimiter avec le corps tubulaire métallique, une enceinte étanche de refroidissement, communiquant avec une entrée d'un fluide de refroidissement et avec une sortie du fluide de refroidissement.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue avec coupe partielle d'un dispositif d'injection conforme à l'invention, en début de poussée.
La **Figure 2** est une vue d'un détail **« A** » du dispositif d'injection illustré à la **Fig. 1****.**
La **Figure 3** est une vue en perspective du dispositif d'injection en fin de poussée.
La **Figure 4** est une vue d'un détail « B » du dispositif illustré à la **Fig. 3****.**
La **Figure 5** est une vue en coupe partielle montrant un détail caractéristique d'une autre variante de réalisation d'un dispositif conforme à l'invention.
La **Figure 6** illustre une vue en coupe partielle montrant une variante de réalisation du dispositif d'injection conforme à l'invention.

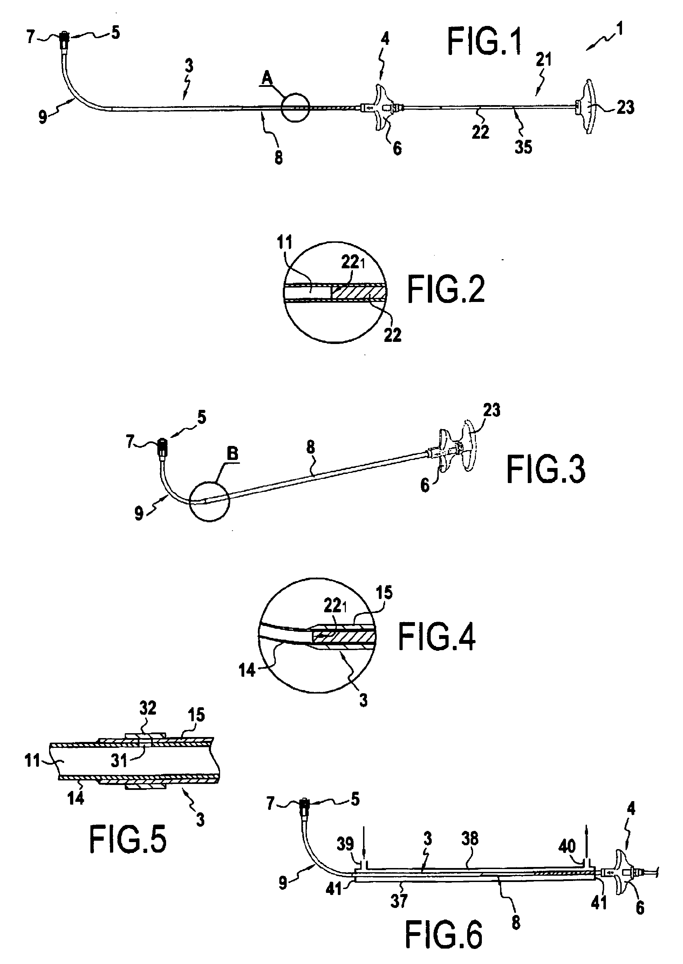

Tel que cela ressort plus précisément des **Fig. 1** à **4****,** l'objet de l'invention concerne un dispositif **1** permettant d'assurer l'injection dans l'organisme, d'un fluide visqueux au sens général. A titre d'exemple, le dispositif **1** permet l'injection en tant que fluide visqueux, de substituts osseux, de ciments osseux ou de tout produit de remplissage intratissulaire.

Le dispositif d'injection **1** comporte un corps tubulaire métallique **3** comportant une première extrémité **4** et une deuxième extrémité **5** opposée à la première. La première extrémité **4** est pourvue d'un organe de préhension **6** rapporté sur le corps tubulaire **3** et réalisé par exemple en matière plastique. Par exemple, l'organe de préhension **6** présente deux branches s'étendant de part et d'autre du corps tubulaire métallique **3** pour permettre l'appui sur sa face avant, des doigts de l'opérateur. La seconde extrémité **5** du corps tubulaire **3** est pourvue d'un raccord **7** permettant la fixation étanche d'un vecteur d'injection transtissulaire de tout type connu en soi tel qu'un trocart ou analogue. Le raccord **7** est réalisé de toute manière appropriée et peut comporter un collier rotatif muni d'un filetage interne pour assurer l'assemblage temporaire étanche avec le vecteur d'injection.

Le corps tubulaire métallique **3** comporte à partir de la première extrémité, une partie rigide **8** et à partir de la seconde extrémité **5,** une partie flexible **9** qui se raccorde à la partie rigide **8.** En d'autres termes, la partie rigide **8** et la partie flexible **9** sont contiguës pour former ensemble le corps tubulaire métallique **3.** Il doit donc être considéré que le corps tubulaire **3** est monobloc ou continu de la première extrémité **4** à la deuxième extrémité **5.** Le corps tubulaire **3** ne présente donc pas de raccord notamment entre la partie rigide **8** et la partie flexible **9** comme peut, par exemple, le présenter la structure tubulaire d'une seringue comportant un corps rigide sur lequel est monté de façon amovible, un prolongateur flexible. La partie rigide **8** et la partie flexible **9** sont liées ensemble de façon définitive ou sans raccord pour former une seule pièce.

Le corps tubulaire **3** délimite intérieurement un canal, un logement ou un alésage interne **11,** de passage pour le fluide visqueux de la première extrémité **4** jusqu'à la deuxième extrémité **5.**

Selon une première variante préférée de réalisation, l'alésage interne **11** du corps tubulaire métallique **3** présente un diamètre sensiblement constant sur toute sa longueur. Par exemple, le corps tubulaire métallique **3** et l'alésage **11** présentent une section droite transversale circulaire. Par exemple, l'alésage **11** du corps tubulaire présente un diamètre compris entre 0,5 mm et 10 mm et de préférence de l'ordre de 3 mm.

Dans l'exemple de réalisation illustré et tel que cela ressort plus précisément de la **Fig. 4****,** le corps tubulaire métallique **3** comporte une chemise flexible métallique **14** s'étendant d'une extrémité à l'autre du corps tubulaire **3** en délimitant l'alésage interne **11** du corps tubulaire. Un tube métallique rigide **15** est fixé par tout moyen approprié sur une partie de la chemise flexible **14** de manière à former la partie rigide **8** du corps tubulaire métallique **3.** Ainsi, une partie de la chemise flexible **14** s'étend en dehors du tube rigide **15** de manière à former la partie flexible **9.** Avantageusement, la partie rigide **8** du corps tubulaire **3** est droite tandis que la partie flexible **9** est courbe ou cintrée de sorte que l'extrémité **7** du corps tubulaire métallique **3** s'étend selon une direction sensiblement perpendiculaire à la direction d'extension de la partie rigide **8.** Bien entendu, la partie flexible **9** peut être déformée afin de donner une angulation différente de celle de **90°** représentée sur les dessins. Par exemple, la chemise flexible métallique **14** et le tube métallique rigide **15** sont réalisés en acier inoxydable de qualité appropriée.

Le dispositif d'injection **1** comporte également un piston de poussée **21** destiné à être engagé dans le corps tubulaire métallique **3** afin d'assurer la poussée d'une dose de fluide visqueux. Le piston de poussée **21** comporte une tige métallique **22** munie à une première extrémité d'un organe de poussée **23** se présentant sous la forme d'une poignée à deux branches s'étendant de part et d'autre de la tige métallique. Selon une variante préférée de réalisation, l'organe de préhension **6** et l'organe de poussée 23 comportent des moyens complémentaires d'assemblage de manière à assurer en fin de course du piston de poussée **21,** un verrouillage du piston de poussée par rapport au corps tubulaire métallique **3.** En d'autres termes, ces moyens de verrouillage permettent en fin de course de poussée, d'assurer le blocage du piston de poussée **21** par rapport au corps **3** par exemple par un mouvement de rotation relatif entre le piston de poussée **21** et le corps tubulaire **3.**

Avantageusement, la tige métallique **22** présente une section droite transversale constante sur toute sa longueur et complémentaire à celle du logement **11** du corps tubulaire métallique **3.** La section droite transversale de la tige métallique **22** est égale, au jeu de fonctionnement près, à la section droite transversale de l'alésage interne **11.** Dans l'exemple de réalisation illustré, les sections droites transversales de la tige métallique **22** et de l'alésage interne **11** sont circulaires, de même valeur, au jeu de fonctionnement près. En d'autres termes, le diamètre de la tige métallique **22** est égal au jeu de fonctionnement près, au diamètre de l'alésage **11** du corps tubulaire **3** dans lequel la tige coulisse. Selon une caractéristique de l'invention, la tige métallique **22** est pleine sur toute sa longueur comme cela apparaît clairement sur les dessins.

La tige métallique **22** présente une longueur inférieure à la longueur de la partie rigide **8** du corps tubulaire métallique. La tige métallique **22** présente à sa seconde extrémité opposée de la première extrémité, une face transversale **22₁** plane et pleine pour assurer la poussée pour le fluide visqueux. En d'autres termes, le fluide visqueux est poussé directement par la face transversale d'extrémité plane **22₁** de la tige métallique **22.** Avantageusement, la tige métallique **22** comporte une face transversale plane de poussée dont la surface est comprise entre 0,2 et 80 mm² et de préférence de l'ordre de 10 mm². Par exemple, la tige métallique **22** possède une longueur comprise entre 5 cm et 50 cm et se trouve réalisée en acier inoxydable de qualité appropriée.

Il est à noter que le corps tubulaire métallique **3** est adapté pour contenir une dose de fluide visqueux à injecter, par exemple de l'ordre de 2,5 cm³. A cet égard, le rapport entre la longueur et le diamètre de la tige métallique **22** est adapté pour réduire la force d'injection tout en permettant injection d'une dose de fluide visqueux en une seule opération de poussée.

Selon une autre caractéristique avantageuse de réalisation, le corps tubulaire métallique **3** comporte une partie flexible **9** dont l'alésage interne **11** possède un diamètre interne qui est supérieur au plus de 30 % par rapport au diamètre interne du vecteur injection transtissulaire. En d'autres termes, la partie flexible **9** présente à sa seconde extrémité, un diamètre interne au plus supérieur de 30 % au diamètre interne du vecteur d'injection transtissulaire de manière à réduire les phénomènes de résistance à l'écoulement du fluide visqueux.

Dans l'exemple illustré, le tube rigide **15** est fixé autour d'une chemise flexible **14** s'étendant sur toute la longueur du tube rigide **15.** Bien entendu, il peut être envisagé une variante de réalisation dans laquelle le corps tubulaire métallique monobloc **3** est réalisé par l'assemblage bout à bout du tube rigide **15** et de la chemise flexible **14.** L'assemblage du tube rigide 15 et de la chemise flexible **14** est réalisé de manière à obtenir un corps tubulaire monobloc non démontable, comme explicité ci-dessus. Il est à noter que selon cette variante de réalisation, la chemise flexible **14** ne s'étend pas sur toute la longueur du tube rigide **15.** Le tube rigide **15** délimite ainsi par son diamètre interne le volume du fluide visqueux à injecter. Selon cette variante de réalisation, le diamètre interne du tube rigide 15 peut être légèrement supérieur au diamètre interne de la partie flexible 9 de manière à définir un volume correspondant à la dose de fluide visqueux à injecter.

Il ressort de la description qui précède que le corps tubulaire **3** et le piston de poussée **21** sont réalisés en un matériau métallique permettant de conférer au dispositif une rigidité ou une résistance à pression élevée et une absence de réaction vis-à-vis du fluide visqueux injecté. Par ailleurs, le dispositif est dimensionné pour réduire la force à exercer pour l'injection tout en présentant un encombrement limité et une adaptation géométrique par rapport à l'orientation du vecteur d'injection. De plus, la réalisation du dispositif d'injection sous une forme allongée permet de déporter l'action de poussée par rapport au vecteur d injection à proximité duquel sont installés divers équipements tels que des systèmes de visualisation ou d'imagerie générant notamment des radiations ionisantes. Enfin, le dispositif permet d'augmenter considérablement le temps pendant lequel le fluide peut être injecté avant sa polymérisation.

La **Fig. 5** illustre une autre variante de réalisation d'un dispositif d'injection **1** conforme à l'invention. Selon cette variante de réalisation, le corps tubulaire comporte un orifice **31** muni d'un organe de sureté **32** qui est adapté pour libérer l'orifce **31** lorsque la pression à l'intérieur du corps tubulaire **3** atteint une valeur déterminée. De préférence, l'orifice **31** est aménagé sur la partie rigide **8** à proximité de la partie flexible **9.**

Selon une variante préférée de réalisation, cet organe de sureté **32** qui agit comme une soupape de sureté ou de surpression est réalisé sous la forme d'un manchon déformable monté sur le corps tubulaire **3** en fermant l'orifice **31.** Lorsqu'une surpression intervient à intérieur du corps **3,** le manchon **32** se déforme pour permettre l'ouverture de l'orifice **31** pour la mise à l'air du corps **3.** Selon une variante de réalisation, le manchon **32** est monté mobile en translation sur le corps tubulaire **3** de manière à permettre de dégager l'orifice **31** afin de purger le corps tubulaire. Ainsi, il peut être possible de dégager la tige de poussée **22** du corps tubulaire de manière à introduire dans le corps **3,** une dose de fluide visqueux destinée à être poussée par ladite tige **22** afin de compléter la dose injectée.

Selon une variante préférée de réalisation, la tige métallique **22** comporte des indicateurs **35** de volume du fluide injecté, réalisés par exemple par des graduations aménagées sur la tige métallique **22**.

La **Fig. 6** illustre une autre variante de réalisation du dispositif d'injection conforme à l'invention visant à contrôler la température du fluide visqueux et par suite sa vitesse de polymérisation. Selon cette variante de réalisation, le dispositif d'injection **1** comporte une enveloppe tubulaire **37** entourant à distance le corps tubulaire métallique **3** sur au moins une partie de sa longueur pour délimiter avec le corps tubulaire **3,** une enceinte **38** contenant un fluide de refroidissement. Cette enceinte **38** communique avec une entrée **39** et une sortie **40** pour le fluide de refroidissement. De préférence, l'enveloppe tubulaire **37** s'étend au plus le long de la partie rigide **8** du corps tubulaire **3.** Cette enveloppe tubulaire **37** est fixée de manière étanche sur le corps tubulaire **3** à l'aide de parois d'extrémité **41.** L'entrée **39** et la sortie **40** communiquent avec un circuit de circulation pour le fluide de refroidissement. Un tel circuit de circulation non représenté mais connu en soi est équipé de moyens permettant de contrôler la température du fluide de refroidissement.

L'invention n'est pas limitée aux exemples décrits et représentés car diverses modifications peuvent y être apportées sans sortir du cadre des revendications.

## Revendications

1. Dispositif pour injection dans l'organisme d'un fluide visqueux, comportant :
- une structure tubulaire délimitant, entre une première extrémité **(4)** d'une deuxième extrémité **(5)**, , un alésage interne **(11)** de passage pour le fluide visqueux, comportant à partir de la première extrémité **(4)** équipée d'un organe de préhension **(6)**, une partie rigide (**8**) et à partir de la deuxième extrémité **(5)**, une partie fléxible **(9)** constigüe à la partie rigide (8)
- un piston de poussée **(21)** engagée dans l'alésage interne **(11),** et équipé à une première extrémité d'un organe de poussée **(23)** et présentant une longueur inférieure à la longueur de la partie rigide **(8),**
- la structure tubulaire étant un corps tubulaire métallique monobloc **(3)** s'étendant de la première extrémité **(4) à** la seconde extrémité **(5)** munie d'un raccord **(7)** pour la fixation étanche d'un vecteur d'injection transtissulaire,
- le piston de poussée **(21)** comportant une tige métallique **(22)** présentant une section droite transversale constante sur toute sa longueur et complémentaire au jeu de fonctionnement près, à celle de l'alésage **(11)** du corps tubulaire métallique **(3),** la tige métallique **(22)** présentant à sa seconde extrémité, une face transversale plane **(22₁)** de poussée pour le fluide visqueux.

2. Dispositif d'injection selon la revendication 1, **caractérisé en ce que** la tige métallique **(22)** comporte des indicateurs **(35)** de volume du fluide injecté.

3. Dispositif d'injection selon la revendication 1 ou 2, **caractérisé en ce que** la tige métallique **(22)** comporte une face transversale plane de poussée de surface comprise entre 0,5 et 80 mm² et par exemple de l'ordre de 10 mm².

4. Dispositif d'injection selon la revendication 1, **caractérisé en ce que** le corps tubulaire métallique **(3)** comporte une partie flexible **(9)** dont le diamètre interne est supérieur au plus de 30 % par rapport au diamètre interne du vecteur d'injection transtissulaire.

5. Dispositif d'injection selon la revendication 1 ou 4, **caractérisé en ce que** le corps tubulaire métallique **(3)** présente un diamètre interne sensiblement constant sur toute sa longueur.

6. Dispositif d'injection selon la revendication 1 ou 5, **caractérisé en ce que** le corps tubulaire **(3)** comporte un tube rigide **(15)** apte à former la partie rigide du corps tubulaire, le tube rigide **(15)** étant fixé autour d'une chemise flexible **(14)** dont une partie s'étend en dehors du tube rigide de manière à former la partie flexible **(9)** du corps tubulaire métallique.

7. Dispositif d'injection selon la revendication 1 ou 4, **caractérisé en ce que** le corps tubulaire **(3)** comporte un tube flexible sur l'extrémité duquel est emmanché un tube rigide dont le diamètre interne détermine le volume du fluide injecté.

8. Dispositif d'injection selon l'une des revendications 1 à 7, **caractérisé en ce que** le corps tubulaire **(3)** comporte un orifice **(31)** muni d'un organe de sureté **(32).**

9. Dispositif selon la revendication 8, **caractérisé en ce que** l'organe de sureté (32) est monté pour dégager l'orifice **(31)** afin de purger le corps tubulaire.

10. Dispositif d'injection selon l'une des revendications 1 à 9, **caractérisé en ce que** le corps tubulaire **(3)** présente une partie rigide droite **(8)** et une partie flexible courbe **(9).**

11. Dispositif d'injection selon l'une des revendications 1 à 10, **caractérisé en ce qu'**il comporte une enveloppe tubulaire **(37)** entourant concentriquement le corps tubulaire métallique **(3)** sur au moins une partie de sa longueur pour délimiter avec le corps tubulaire métallique (3), une enceinte **(38)** étanche de refroidissement, communiquant avec une entrée **(39)** d'un fluide de refroidissement et avec une sortie **(40)** du fluide de refroidissement.

## Claims

1. A device for injecting a viscous fluid into the body, including:
- a tubular structure defining, between a first end **(4)** and a second end **(5),** an inner bore **(11)** for passage of the viscous fluid, including, from the first end **(4)** equipped with a gripping member **(6),** a rigid portion (8) and from the second end (5), a flexible portion (9)adjacent to the rigid portion **(8),**
- a pushing piston **(21)** engaged in the inner bore **(11),** and equipped at a first end with a pushing member **(23)** and having a length smaller than the length of the rigid portion **(8),** the tubular structure being a single-piece metal tubular body **(3)** extending from the first end **(4)** to the second end **(5)** provided with a connector **(7)** for sealable fastening of a trans-tissue injection vector,
- the pushing piston **(21)** having a metal rod **(22)** having a straight transverse section that is constant over its entire length and complementary, while allowing for operational clearance, with that of the bore **(11)** of the metal tubular body (3), the metal rod **(22)** having, at its second end, a planar transverse face **(22₁)** for pushing the viscous fluid.

2. The injection device according to claim 1, **characterized in that** the metal rod **(22)** includes indicators **(35)** of the volume of injected fluid.

3. The injection device according to claim 1 or 2, **characterized in that** the metal rod **(22)** includes a planar transverse pushing face with a surface between 0.5 and 80 mm², and for example in the vicinity of 10 mm².

4. The injection device according to claim 1, **characterized in that** the metal tubular body (3) includes a flexible portion **(9)** whereof the inner diameter is no more than 30% larger than the inner diameter of the trans-tissue injection vector.

5. The injection device according to claim 1 or 4, **characterized in that** the metal tubular body (3) has an inner diameter essentially constant over its entire length.

6. The injection device according to claim 1 or 5, **characterized in that** the tubular body (3) includes a rigid rube **(15)** capable of forming the rigid portion of the tubular body, the rigid tube **(15)** being fastened around a flexible lining **(14)** whereof one portion extends outside the rigid tube so as to form the flexible portion **(9)** of the metal tubular body.

7. The injection device according to claim 1 or 4, **characterized in that** the tubular body **(3)** includes a flexible tube on the end of which is fitted a rigid tube whereof the inner diameter determines the volume of the injected fluid.

8. The injection device according to one of claims 1 to 7, **characterized in that** the tubular body **(3)** includes an orifice **(31)** provided with a safety member **(32).**

9. The device according to claim 8, **characterized in that** the safety member **(32)** is mounted to release the orifice **(31)** in order to purge the tubular body.

10. The injection device according to one of claims 1 to 9, **characterized in that** the tubular body **(3)** has a straight rigid portion **(8)** and a curved flexible portion **(9).**

11. The injection device according to one of claims 1 to 10, **characterized in that** it includes a tubular jacket **(37)** concentrically surrounding the metal tubular body (3) on at least part of its length to define, with the metal tubular body **(3),** a sealed cooling enclosure **(38),** communicating with an inlet **(39)** of a coolant and with an outlet **(40)** of the coolant.

## Patentansprüche

1. Vorrichtung zum Injizieren eines viskosen Fluids in den Organismus, umfassend:
- eine zwischen einem ersten Ende (4) und einem zweiten Ende (5) eine Durchgangsinnenbohrung (11) für das viskose Fluid begrenzende röhrenförmige Struktur, die ausgehend von dem ersten Ende (4), das mit einem Greiforgan (6) ausgestattet ist, einen starren Teil (8) und ausgehend von dem zweiten Ende (5) einen an den starren Teil (8) angrenzenden flexiblen Teil (9) umfaßt,
- einen Schubkolben (21), der in die Innenbohrung (11) eingesteckt ist und der an einem ersten Ende mit einem Schiebeorgan (23) versehen ist und eine Länge aufweist, die geringer als die Länge des starren Teils (8) ist,
- wobei die röhrenförmige Struktur ein einteiliger röhrenförmiger Metallkörper (3) ist, der sich von dem ersten Ende (4) zum zweiten Ende (5), das mit einem Anschluß (7) für das dichte Befestigen eines Trägers für die Injektion durch das Gewebe versehen ist, erstreckt,
- wobei der Schubkolben (21) eine Metallstange (22) umfaßt, die einen Querschnitt aufweist, der über ihre gesamte Länge konstant und bis auf das Betriebsspiel zu demjenigen der Bohrung (11) des röhrenförmigen Metallkörpers (3) komplementär ist, wobei die Metallstange (22) an ihrem zweiten Ende eine ebene Schubquerfläche (22₁) für das viskose Fluid aufweist.

2. Injektionsvorrichtung nach Anspruch 1, **dadurch gekennzeichnet, daß** die Metallstange (22) Anzeiger (35) für die Menge des injizierten Fluids umfaßt.

3. Injektionsvorrichtung nach Anspruch 1 oder 2, **dadurch gekennzeichnet, daß** die Metallstange (22) eine ebene Schubquerfläche mit einer Fläche zwischen 0,5 und 80 mm² und beispielsweise in der Größenordnung von 10 mm² umfaßt.

4. Injektionsvorrichtung nach Anspruch 1, **dadurch gekennzeichnet, daß** der röhrenförmige Metallkörper (3) einen flexiblen Teil (9) umfaßt, dessen Innendurchmesser im Vergleich zum Innendurchmesser des Trägers für die Injektion durch das Gewebe höchstens um 30 % größer ist.

5. Injektionsvorrichtung nach Anspruch 1 oder 4, **dadurch gekennzeichnet, daß** der röhrenförmige Metallkörper (3) einen im wesentlichen konstanten Innendurchmesser über seine gesamte Länge aufweist.

6. Injektionsvorrichtung nach Anspruch 1 oder 5, **dadurch gekennzeichnet, daß** der röhrenförmige Körper (3) eine starre Röhre (15) umfaßt, die geeignet ist, den starren Teil des röhrenförmigen Körpers zu bilden, wobei die starre Röhre (15) um einen flexiblen Mantel (14) herum befestigt ist, wovon ein Teil außerhalb der starren Röhre verläuft, um den flexiblen Teil (9) des röhrenförmigen Metallkörpers zu bilden.

7. Injektionsvorrichtung nach Anspruch 1 oder 4, **dadurch gekennzeichnet, daß** der röhrenförmige Körper (3) eine flexible Röhre umfaßt, an deren Ende eine starre Röhre angesetzt ist, deren Innendurchmesser die Menge des injizierten Fluids bestimmt.

8. Injektionsvorrichtung nach einem der Ansprüche 1 bis 7, **dadurch gekennzeichnet, daß** der röhrenförmige Körper (3) eine Öffnung (31) umfaßt, die mit einem Sicherheitsorgan (32) ausgestattet ist.

9. Vorrichtung nach Anspruch 8, **dadurch gekennzeichnet, daß** das Sicherheitsorgan (32) angebracht ist, um die Öffnung (31) für das Entleeren des röhrenförmigen Körpers freizugeben.

10. Injektionsvorrichtung nach einem der Ansprüche 1 bis 9, **dadurch gekennzeichnet, daß** der röhrenförmige Körper (3) einen geraden starren Teil (8) und einen gekrümmten flexiblen Teil (9) umfaßt.

11. Injektionsvorrichtung nach einem der Ansprüche 1 bis 10, **dadurch gekennzeichnet, daß** sie eine röhrenförmige Hülle (37) umfaßt, die den röhrenförmigen Metallkörper (3) über wenigstens einen Teil seiner Länge konzentrisch umschließt, um mit dem röhrenförmigen Metallkörper (3) einen dichten Kühlraum (38) zu bilden, der mit einem Einlaß (39) für ein Kühlfluid und mit einem Auslaß (40) des Kühlfluids in Verbindung ist.
